# EUROPEAN PATENT SPECIFICATION

(11) **EP 2 305 126 B1**
(45) Date of publication and mention of the grant of the patent: **22.08.2012**
(21) Application number: 10251694.5
(22) Date of filing: 30.09.2010
(51) Int. Cl.: A61B 17/00, A61B 17/08, A61B 17/064

(54) **Wound closure device including pivotable claws**
Wundverschlussvorrichtung mit schwenkbaren Greifern
Dispositif de fermeture de plaie incluant des griffes pivotantes

(30) Priority: 01.10.2009 US 247639 P; 22.09.2010 US 887890
(43) Date of publication of application: 06.04.2011
(73) Proprietor: Tyco Healthcare Group LP, New Haven, CT 06511 (US)
(72) Inventor: Viola, Frank J., Sandy Hook, CT 06482 (US)
(74) Representative: Soames, Candida Jane

(56) References cited:
- US-A- 4 733 664
- US-A1- 2004 097 982
- US-A1- 2005 288 707
- US-A1- 2009 143 808

## Description

### BACKGROUND

### Technical Field

The present disclosure relates to a wound closure device, and more particularly, to a wound closure device having pivotable claws for closing a wound.

### Description of Related Art

Puncture wounds, wounds that pierce through tissue, may result from trauma or may be intentionally created in order to provide access to a body cavity during surgical procedures. During endoscopic surgical procedures, for example, a trocar device is utilized to puncture the peritoneum to provide an access port by way of a cannula through the abdominal wall. Generally, a trocar and/or a cannula is placed through the abdominal wall for introduction of surgical instrumentation which is necessary to carry out the surgical procedure. In this manner, the surgeon may introduce a surgical instrument such as a grasper, scissor, clip applier, stapler or any other surgical instrument which may be necessary during the particular surgical procedure. Once the procedure is complete, it is necessary to close the wound in order to protect against hernia, adhesions, and other undesirable conditions.

US2004/0097982, on which the precharacterising portion of claim 1 is based, discloses a wound closure device having a plurality of claws which are pivotally mounted to a housing and are pivotable between a first open position and a second closed position. In the closed position, the jaws are locked in place by a ratchet mechanism.

### SUMMARY

Accordingly, the present invention provides a wound closure device according to claim 1.

In embodiments, the plurality of claws is pivotably mounted to a bridge disposed therebetween. The shaft may be disposed in mechanical cooperation with the claw assembly such that the shaft separates from the claw assembly upon the mechanical disengagement therefrom. In embodiments, one or both of the claw assembly and the shaft are biodegradable.

### BRIEF DESCRIPTION OF THE DRAWINGS

The above and other aspects, features, and advantages of the present disclosure will become more apparent in light of the following detailed description when taken in conjunction with the accompanying drawings in which:

Fig. 1 is a perspective view of a wound closure device in a first position in accordance with the present invention;

Fig. 2 is a perspective view of the wound closure device of Fig. 1 in a second position;

Fig. 3 is a perspective view of another embodiment of a wound closure device in a first position not in accordance with the present invention; and

Fig. 4 is a perspective view of the wound closure device of Fig. 3 in a second position.

### DETAILED DESCRIPTION

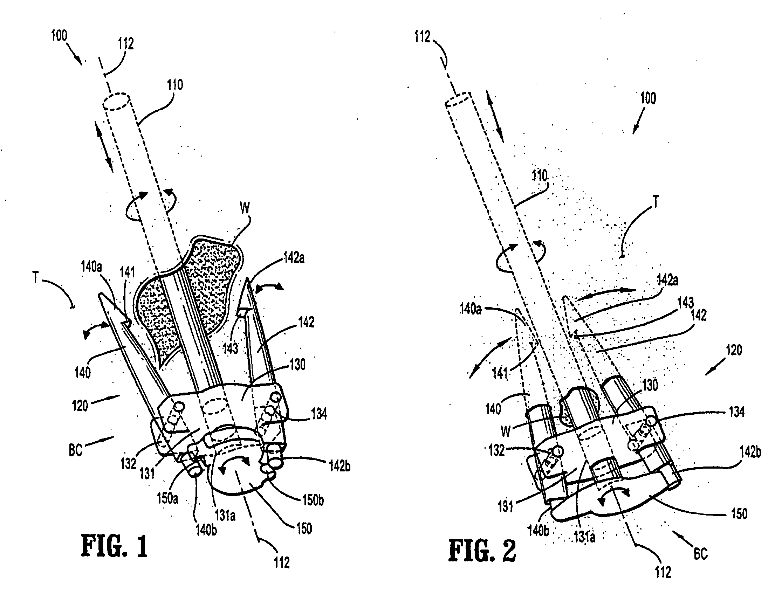

A particular embodiment of the present disclosure will be described herein with reference to the accompanying figs. 1-2. As shown in the drawings and as described throughout the following description, and as is traditional when referring to relative positioning on an object, the terms "proximal" and "trailing" may be employed interchangeably, and should be understood as referring to the portion of a structure that is closer to a clinician during proper use. The terms "distal" and "leading" may also be employed interchangeably, and should be understood as referring to the portion of a structure that is farther from the clinician during proper use. In the following description, well-known functions or constructions are not described in detail to avoid obscuring the present disclosure in unnecessary detail.

Referring now in specific detail to the drawings, in which like numbers identify similar or identical elements, Figs. 1 and 2 illustrate an embodiment of a wound closure device in accordance with the invention and is generally designated 100. The wound closure device 100 includes a shaft 110 and a claw assembly 120. The claw assembly 120 is operably associated with the shaft 110 and includes a housing 130, claws 140, 142, and a cam 150. The housing 130 includes a housing body 131 and pivot pins 132, 134. The housing 130 is mounted to the distal end of the shaft 110 via shaft bore 131 a extending through the housing 130 along a longitudinal axis 112 of the shaft 110. The claws 140, 142 are pivotally mounted to the housing 130 via pivot pins 132, 134 and include tips 140a, 142a at the proximal ends thereof for engaging tissue "T" and extensions 140b, 142b at the distal end thereof. The tips 140a, 142a have one or more barbs 141, 143 extending therefrom. The extensions 140b, 142b are adapted to lockingly engage the cam 150 as described below. The claws 140, 142 are positionable between a first position (Fig. 1) and a second position in which the claws 140, 142 are lockingly engaged with the tissue "T" adjacent a wound "W" for maintaining the wound "W" in a closed position (Fig. 2). The cam 150 is supported for rotation on the distal end of the shaft 110. The cam 150 rotates in response to rotation of the shaft 110. As the cam 150 rotates, the claws 140, 142 pivot between the first and second positions. The cam 150 may include one or more notches 150a, 150b adapted to engage the extensions 140b, 142b of the claws 140, 142 and are adapted to lock each claw 140, 142 in the second position when the extensions 140b, 142b of the claws 140, 142 are engaged with the one or more notches 150a, 150b.

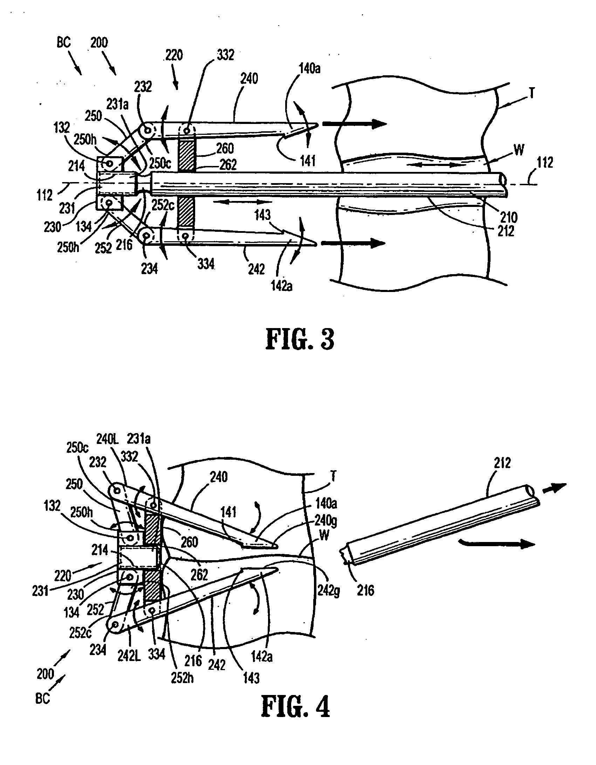

The shaft 110 may be adapted to separate from the claw assembly 120 in the second position such that the claw assembly 120 remains attached to the tissue "T" and within the body cavity "BC" so that the wound "W" may remain closed for effective healing thereof. The shaft 110 may include a frangible portion (see Fig. 4) adapted to fracture in the second position. In embodiments, a portion of the shaft 110 and/or the claw assembly 120 is biodegradable, allowing the wound "W" to heal as they are absorbed into the body. Also, the shaft 110 may be formed of absorbable material and cut near the surface of skin.

In use, the claw assembly 120 is inserted into the wound "W" such that the claws 140, 142 are disposed within a body cavity "BC" of a patient. The claws 140, 142 are then positioned within internal tissue "T" adjacent the wound "W" by proximally translating the shaft 110 such that each claw 140, 142 is fixedly engaged within the tissue "T." Upon rotation of the shaft 110, the cam 150 rotates each claw 140, 142 between the first and the second position. The notches 150a, 150b of the cam 150 engage extensions 140b, 142b of the claws 140, 142 such that the claws 140, 142 are lockingly engaged in the second position. The shaft 110 may then be removed from the claw assembly 120 and out from the wound "W." The shaft 110 may be mechanically disengaged (e.g., unscrewed) for removal thereof from the claw assembly 120. In embodiments, as discussed above, the shaft may have a frangible portion adapted to fracture upon the application of force in order to remove a portion of the shaft proximal the frangible portion (see Fig. 4) from the claw assembly 120.

Referring now to Figs. 3 and 4, another wound disclosure device 200, not according to the claimed invention, includes a shaft 210 and a claw assembly 220. The shaft 210 includes a proximal portion 212, a distal portion 214, and a frangible portion 216 sandwiched therebetween. The claw assembly 220 is operably associated with shaft 210 and includes a housing 230, claws 240, 242, linkages 250, 252, and a bridge 260.

With continued reference to Figs. 3 and 4, the housing 230 includes a housing body 231 and pivot pins 132, 134. The housing 230 is mounted to the distal end of the shaft 210 via a shaft bore 231a extending through the housing 230 along a longitudinal axis 112 of the shaft 210. The claws 240, 242 are pivotally mounted to the linkages 250, 252 via pivot pins 232, 234 and are pivotally mounted to the bridge 260 via pivot pins 332, 334. The bridge 260 includes a bore 262 therethrough for permitting passage of the shaft 210 therethrough. The claws 240, 242 include tips 140a, 142a at the proximal ends thereof for engaging tissue "T." The tips 140a, 142a have one or more barbs 141, 143 extending therefrom. The linkages 250, 252 are pivotally mounted to the housing 230 via pivot pins 132, 134 such that the longitudinal translation of the housing 230 causes linkages 250, 252 and claws 240, 242 to move between the first and second positions. The linkages 250, 252 may be "living" hinges.

The claws 240, 242 are positionable between a first position (Fig. 3) and a second position in which the claws 240, 242 are lockingly engaged with the tissue "T" adjacent a wound "W" for maintaining the wound "W" in a closed position (Fig. 4). The housing 230 axially translates along the longitudinal axis 112 of the shaft 210 when the claws 240, 242 move between the first and second positions. The housing 230 may be locked in a proximal-most position (Fig. 4) when the claws 240, 242 are in the second position. In the second position, the frangible portion 216 of the shaft 210 may be at least partially disposed within the bore 262 of the bridge 260. The frangible portion 216 of the shaft 210 is configured to break or tear upon the application of a predetermined shear and/or axial force thereto. As such, the proximal portion 212 of the shaft 210 may be separated from the claw assembly 220 and out from within the wound "W" while the claw assembly 220 and the distal portion 214 of the shaft 210 remain attached to the tissue "T" and within the body cavity "BC" so that the wound "W" may remain closed for effective healing thereof. The distal portion 214 of the shaft 210 and the claw assembly 220 may be biodegradable, allowing the wound "W" to heal as they are absorbed into the body.

In use, the claw assembly 220 is inserted into the wound "W" such that the claws 240, 242 are disposed within a body cavity "BC" of a patient. The claws 240, 242 are then positioned within internal tissue "T" adjacent the wound "W" by proximally translating the shaft 210 such that each claw 240, 242 is fixedly engaged within the tissue "T." Upon further longitudinal translation of the shaft 210, the linkages 250, 252 pivot proximally with the housing body 231 toward the bridge 260 via pivot pins 132, 134 on housing ends 250h, 252h thereof and

While one embodiment of the disclosure has been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. A wound closure device (100), comprising:
a shaft (110) defining a longitudinal axis; and
a claw assembly (120) operably associated with the shaft and including a housing (130) and a plurality of claws (140, 142) pivotally mounted to the housing and positionable between a first position and a second position in which the claws are locked and adapted to be engaged with tissue adjacent a wound for maintaining the wound in a closed position to promote healing thereof, wherein the shaft is adapted to separate from the claw assembly in the second position;
**characterised by**
at least one cam (150) supported for rotation on the distal end of the shaft such that the at least one cam rotates in response to rotation of the shaft, wherein the rotation of the at least one cam pivots the claws between the first and second positions and wherein the at least one cam includes a plurality of notches (150a, 150b), each adapted to lock one claw in the second position when the claw is engaged thereto.

2. The wound closure device of claim 1, wherein the plurality of claws is pivotably mounted to a bridge disposed therebetween.

3. The wound closure device of claim 1 or 2, wherein the shaft includes a frangible portion adapted to fracture in the second position.

4. The wound closure device of claim 1 or 2, wherein the shaft is disposed in mechanical cooperation with the claw assembly such that the shaft separates from the claw assembly upon the mechanical disengagement therefrom.

5. The wound closure device of any preceding claim, wherein at least one of the claw assembly and the shaft is biodegradable.

## Patentansprüche

1. Wundverschlussvorrichtung (100) enthaltend:
einen Schaft (110), der eine Längsachse definiert; und
eine Greiferanordnung (120), funktionell mit dem Schaft verbunden, mit einem Gehäuse (130) und einer Mehrzahl von Greifern (140, 142), schwenkbar an das Gehäuse montiert und zwischen einer ersten Position und einer zweiten Position positionierbar, in der die Greifer gesperrt und konstruiert sind, um auf das Gewebe neben einer Wunde geführt zu werden und die Wunde in einer geschlossenen Position zu halten, um die Heilung derselben zu fördern, wobei der Schaft konstruiert ist, um in der zweiten Position der Greiferanordnung gelöst zu werden;
**gekennzeichnet durch**
mindestens einen Nocken (150), geeignet zur Drehung am distalen Ende des Schafts, damit sich mindestens ein Nocken in Reaktion auf die Drehung des Schafts dreht, wobei die Drehung von mindestens einem der Nocken die Greifer zwischen der ersten und zweiten Position schwenkten lässt und mindestens einer der Nocken eine Mehrzahl von Kerben (150a, 150b) aufweist, jeweils so konstruiert, um einen Greifer in der zweiten Position zu sperren, wenn der Greifer dorthin geführt wird.

2. Wundverschlussvorrichtung nach Anspruch 1, wobei die Mehrzahl von Greifern schwenkbar an eine dazwischen angeordnete Brücke montiert sind.

3. Wundverschlussvorrichtung nach Anspruch 1 oder 2, wobei der Schaft einen zerbrechlichen Teil aufweist, konstruiert, in der zweiten Position zu brechen.

4. Wundverschlussvorrichtung nach Anspruch 1 oder 2, wobei der Schaft in mechanischer Zusammenwirkung mit der Greiferanordnung angeordnet ist, damit der Schaft bei mechanischer Trennung von der Greiferanordnung gelöst wird.

5. Wundverschlussvorrichtung nach einem beliebigen der vorhergehenden Ansprüche, wobei mindestens eine der Greiferanordnungen und der Schaft biologisch abbaubar sind.

## Revendications

1. Dispositif de fermeture d'une plaie (100), comprenant :
une tige (110) définissant un axe longitudinal ; et
un ensemble de griffes (120), associé de manière opérationnelle à la tige principale et comprenant un boîtier (130) et plusieurs griffes (140, 142) fixées à pivotement au boîtier et positionnables entre une première position et une deuxième position dans laquelle les griffes sont verrouillées et destinées à s'engager avec le tissu adjacent à une plaie pour maintenir la plaie dans une position fermée afin d'en favoriser la cicatrisation, la tige étant destinée à se séparer de l'ensemble de griffes dans la deuxième position ;
**caractérisé par**
au moins une came (150) soutenue à rotation sur l'extrémité distale de la tige de telle sorte qu'une came au moins tourne en réaction à la rotation de la tige, la rotation d'une came au moins faisant pivoter les griffes entre les première et deuxième positions et au moins une came comprenant plusieurs encoches (150a, 150b) destinées chacune à verrouiller une griffe dans la deuxième position lorsque la griffe s'y est engagée.

2. Dispositif de fermeture d'une plaie selon la revendication 1, dans lequel les plusieurs griffes sont fixées à pivotement à un pont disposé dans l'intervalle.

3. Dispositif de fermeture d'une plaie selon l'une des revendications 1 ou 2, dans lequel la tige comprend une partie cassable destinée à se rompre dans la deuxième position.

4. Dispositif de fermeture d'une plaie selon l'une des revendications 1 ou 2, dans lequel la tige est disposée en coopération mécanique avec l'ensemble de griffes de telle sorte que la tige se sépare de l'ensemble de griffes lors de son dégagement mécanique de celui-ci.

5. Dispositif de fermeture d'une plaie selon l'une des revendications précédentes, dans lequel au moins un des éléments, entre l'ensemble de griffes et la tige, est biodégradable.
